(19) **Europäisches Patentamt European Patent Office Office européen des brevets**

(11) **EP 3 500 201 B1**

(12) **EUROPEAN PATENT SPECIFICATION**

(45) Date of publication and mention
of the grant of the patent:
**23.10.2019 Bulletin 2019/43**

(21) Application number: **17757511.5**

(22) Date of filing: **21.08.2017**

(51) Int Cl.:
**A61B 18/20** *(2006.01)* **A61B 18/00** *(2006.01)*

(86) International application number:
**PCT/EP2017/070988**

(87) International publication number:
**WO 2018/033644 (22.02.2018 Gazette 2018/08)**

(54) **A CUTTING ELEMENT FOR A HAIR CUTTING DEVICE**

SCHNEIDEELEMENT FÜR EINE HAARSCHNEIDEVORRICHTUNG

ÉLÉMENT DE COUPE POUR UN DISPOSITIF DE COUPE DE CHEVEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.08.2016 EP 16184989**

(43) Date of publication of application:
**26.06.2019 Bulletin 2019/26**

(73) Proprietor: **Koninklijke Philips N.V.
5656 AE Eindhoven (NL)**

(72) Inventors:
  • **VERHAGEN, Rieko**
    **5656 AE Eindhoven (NL)**
  • **BOURQUIN, Yannyk, Parulian, Julian**
    **5656 AE Eindhoven (NL)**
  • **TER BORCH, Joseph, Petrus, Henricus**
    **5656 AE Eindhoven (NL)**

  • **JUTTE, Petrus, Theodorus**
    **5656 AE Eindhoven (NL)**
  • **THUMMA, Kiran, Kumar**
    **5656 AE Eindhoven (NL)**
  • **MOESKOPS, Bastiaan, Wilhelmus, Maria**
    **5656 AE Eindhoven (NL)**
  • **JOHNSON, Mark, Thomas**
    **5656 AE Eindhoven (NL)**

(74) Representative: **Uittenbroek, Arie Leendert
Philips Intellectual Property & Standards
High Tech Campus 5
5656 AE Eindhoven (NL)**

(56) References cited:
**WO-A1-2014/143670      US-A1- 2008 244 912
US-A1- 2012 123 444      US-A1- 2014 140 091
US-A1- 2015 359 592**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The invention relates to a cutting element for use with a hair cutting device for cutting (e.g. shaving) hair on a body of a subject, and a hair cutting device comprising the same.

BACKGROUND OF THE INVENTION

Shaving devices for cutting or shaving hair on a body of a subject typically make use of one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular, a laser light source is provided that is configured to generate laser light having a wavelength selected to target a predetermined chromophore to effectively cut a hair shaft. A fibre optic is located on a shaving portion of the device that is positioned to receive the laser light from the laser light source at a proximal end, conduct the laser light from the proximal end toward a distal end, and emit the light out of a cutting region of the fibre optic and toward hair when the cutting region is brought in contact with the hair.

SUMMARY OF THE INVENTION

To achieve a close shave, the cutting element of the shaving device (i.e. the fibre optic in the case of the device in WO 2014/143670) needs to be brought very close to, or into contact with, hair to be cut. Since both the fibre optic of the cutting element and the hair to be cut are generally cylindrical in shape, the surface area via which light can couple from the fibre optic to the hair is very small.

In order to increase the efficiency of the energy transfer from the fibre optic to the hair and, hence, to improve the cutting efficiency of the shaving device, the amount of light able to contact the hair from the fibre optic should ideally be increased.

Therefore, there is a need for an improved cutting element and hair cutting device that have an improved cutting effectiveness.

According to a first aspect, there is provided a cutting element for use in a hair cutting device, the cutting element comprising an optical waveguide having a sidewall which is substantially parallel to the longitudinal axis of the optical waveguide, wherein a portion of the sidewall forms a cutting face for contacting hair, wherein the cutting face has a structure comprising a plurality of indentations configured to allow light to couple out through the sidewall to increase the amount of light able to couple to hair. An advantage of a cutting face having such a structure is that out-coupled light is able to couple to hair before the hair comes into contact with the cutting face, and before the effect of frustrated total internal reflection can cut the hair. Thus, the arrangement can cause the cutting of hair to be initiated sooner, thereby improving the cutting efficiency of the cutting element. Furthermore, the out-coupled light that couples to the hair may cause the hair to deform, thereby increasing the area of contact between the hair and the cutting face of the cutting element, and improving the cutting effect resulting from frustrated total internal reflection.

The structure of the cutting face may be such that at least some of the light allowed to couple out through the sidewall is directed back into the optical waveguide. In this way, light is effectively "recycled", and losses of light through out-coupling into the surrounding medium are reduced.

The structure of the cutting face may be configured to increase the numerical aperture of light propagating through the optical waveguide. In some embodiments, the structure of the cutting face may comprise a serrated structure. The numerical aperture of light may be considered to be the same as the maximum possible angle of incidence of the light.

The cutting face may comprise a plurality of indentations formed therein. Each of the plurality of indentations may be configured such that light coupling out through a first wall of the indentation is refracted towards a second wall of the indentation. In this way, out-coupled light is caused to pass through a cavity or recess formed in the indentation, into which a hair may be moved to initiate cutting thereof.

Each of the indentations may comprise one of a wedge-shaped indentation, a V-shaped indentation a cone-shaped indentation, or a channel. Each indentation may be configured to receive at least a portion of a strand of hair within the indentation.

Each indentation may comprise an apex having an apex angle of between approximately 20 and 80 degrees. A refraction angle of light out-coupling from the indentation may be approximately equal to a total internal reflection angle of the optical waveguide.

The structure of the cutting face may comprise a rough surface, such that a portion of the light propagating through the optical waveguide and incident on the cutting face is caused to couple out through the cutting face. The rough surface may comprise a plurality of indentations and protrusions. Each indentation and protrusion may have a respective depth and height less than the wavelength of the light intended to propagate through the optical waveguide. In this way, a very small amount of light is able to couple out from the optical waveguide, which may couple to a hair to initiate cutting or melting.

The optical waveguide may be an optical fibre. The optical fibre may comprise a fused silica or quartz fibre.

The cutting element may further comprise a plurality of protrusions formed on the cutting face of the optical waveguide, the protrusions configured to guide hair towards the cutting face. This helps to ensure that hairs are cut during a single pass of the cutting element, and increases the chance that all hairs will be guided into a desired position (e.g. in an indentation or into an area of out-coupled light) in the cutting face.

According to a second aspect, there is provided a hair cutting device for cutting hair on a body of a subject, the hair cutting device comprising a light source for generating laser light at one or more specific wavelengths corresponding to wavelengths absorbed by one or more chromophores in hair; and a cutting element coupled to the light source to receive laser light. The cutting element may comprise a cutting element as described above.

Other advantageous features will become apparent from the following description.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a block diagram of a hair cutting device according to an embodiment of the invention;
Fig. 2 is a pair of schematic drawings showing different views of an exemplary hair cutting device according to an embodiment of the invention;
Fig. 3 is a graph illustrating the refractive index of hair;
Fig. 4 is an illustration of an optical waveguide cutting element according to a specific embodiment;
Fig. 5 is an illustration of an optical waveguide cutting element according to a specific embodiment;
Fig. 6 is an enlarged view of part of the optical waveguide of Fig. 5;
Fig. 7 is an illustration of an optical waveguide cutting element according to an alternative embodiment; and
Fig. 8 is an illustration of an optical waveguide cutting element according to a further specific embodiment.

DETAILED DESCRIPTION OF THE EMBODIMENTS

As noted above, the present invention provides an improvement in the cutting ability and efficiency of a laser light-based shaving device, for example as described in WO 2014/143670. In particular, it has been recognised that by increasing the amount of light able to couple to hair from the cutting element, the hair can be cut more cleanly and effectively, reducing the need for a user of the shaving device to repeatedly use the shaving device over the same area of his or her skin and, consequently, reducing the risk of pain or irritation of the skin.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

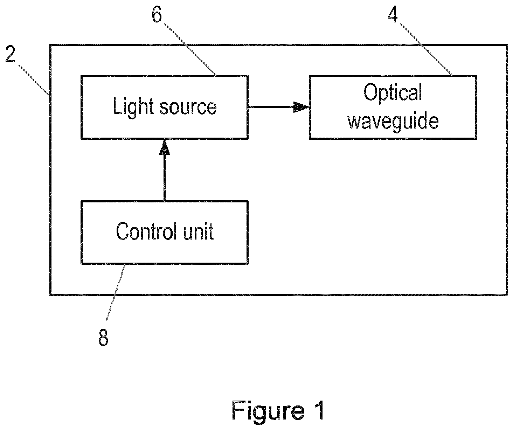

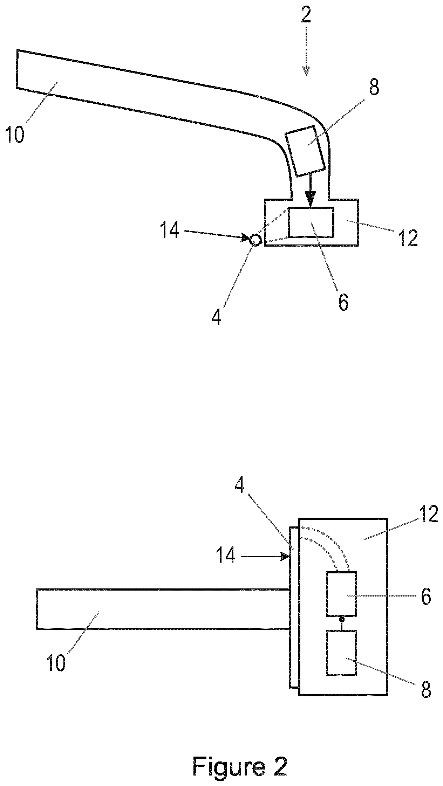

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the sidewall of the optical waveguide 4 (the sidewall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A light source 6 is provided in the hair cutting device 2 that generates laser light at one or more specific wavelengths. The light source 6 is optically coupled to the optical waveguide 4 so that the laser light generated by the light source 6 is coupled into the optical waveguide 4 (and specifically coupled into an end of the optical waveguide 4 so that the laser light propagates through the optical waveguide 4).

The light source 6 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometres) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 6 can be provided that each generate laser light at a respective wavelength, and each light source 6 can be coupled to a respective optical waveguide 4 to provide multiple cutting elements 4 in the device 2.

The hair cutting device 2 also comprises a control unit 8 that controls the operation of the hair cutting device 2, and in particular is connected to the light source 6 to control the activation and deactivation of the light source 6. The control unit 8 may activate and deactivate the light source 6 in response to an input from a user of the hair cutting device 2. The control unit 8 can comprise one or more processors, processing units, multi-core processors or modules that are configured or programmed to control the hair cutting device 2.

As noted above, Fig. 2 shows a hair cutting device 2 that is in the form of a handheld wet razor. Fig. 2 shows a side view and a bottom view of the razor 2. The razor 2 comprises a handle 10 for the subject (or other user of the device 2) to hold, and a head portion 12 that includes the cutting element 4 (optical waveguide/fibre). As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A light source 6 and control unit 8 are shown as being incorporated into the head portion 12 and handle 10 respectively, but it will be appreciated that the positions of these components in the hair cutting device 2 as shown in Fig. 2 is not limiting. Likewise it will be appreciated that the embodiment shown in Fig. 2 is merely an example, and the invention can be incorporated or used in any type of hair cutting device 2 that comprises an optical waveguide cutting element 4 as described herein.

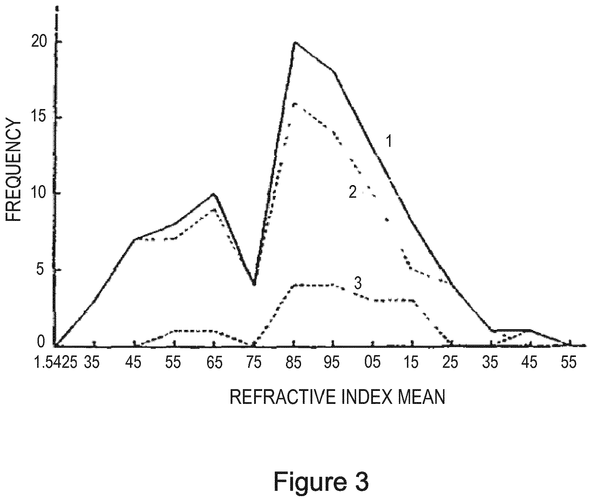

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 should preferably have the same or a lower refractive index than hair at the point at which the hair contacts the optical waveguide 4. Thus, the optical waveguide 4 should preferably have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair. Light may still be able to couple from the optical waveguide 4 into an object (e.g. a hair) brought into contact with the cutting face 14 of the optical waveguide even if the refractive index of the optical waveguide is higher than that of the object, due to a high numerical aperture in the cutting face.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise silica, fluoride glass, phosphate glass, chalcogenide glass, crown glass (such as BK7) and/or crystals (such as yttrium aluminium garnet (YAG) or sapphire).

As noted above, the optical waveguide/fibre 4 and strands of hair to be cut by the cutting device are generally cylindrical in shape, and the optical waveguide and a strand of hair are typically oriented such that they are approximately perpendicular to one another when cutting is taking place. Therefore, the surface area of the optical waveguide 4 that can come into contact with a strand of hair, and therefore transfer energy to the hair to effect cutting of the hair, is very small. Therefore, it has been recognised that structuring the cutting face 14 in such a way that allows some of the light propagating through the optical waveguide 4 to couple out from the optical waveguide into a surrounding medium helps to initiate the melting or cutting of hair.

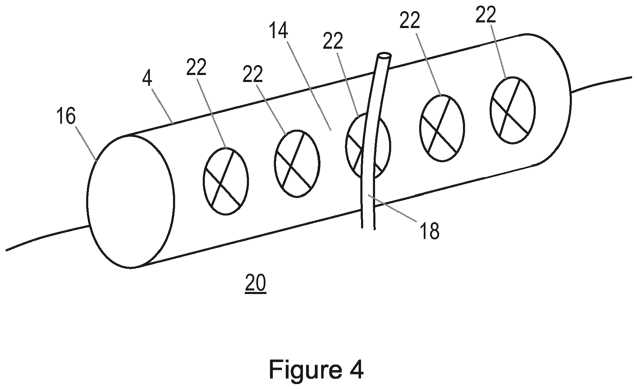

Fig. 4 illustrates an exemplary embodiment of the cutting element 4 (optical waveguide 4) according to the invention. In Fig. 4, only a portion of the optical waveguide 4 part of the hair cutting device 2 is shown, and the optical waveguide 4 is shown in perspective view. No support structure for the optical waveguide 4 is shown.

In Fig. 4, an optical waveguide 4 is shown that has a core 16. In this illustrated embodiment, the optical waveguide 4 does not include any cladding around the core 16. However, it will be appreciated that in some embodiments the optical waveguide 4 can comprise cladding around the core 16, although preferably no cladding is present along the cutting face 14 (and indeed, in some embodiments the cutting face 14 can correspond to those parts of the optical waveguide 4 where there is no cladding).

The optical waveguide 4 is shown in contact with a hair 18 and the skin 20. The portion of the sidewall of the core 16/optical waveguide 4 that is intended to contact hairs during use forms the cutting face 14. As described above, the refractive index of the core 16 is the same or lower than the refractive index of hair.

The cutting face 14 of the optical waveguide 4 includes a structure which, in this embodiment, comprises a plurality of indentations 22. The structure of the cutting face 14 allows light to couple out through the sidewall to increase the amount of light able to couple to hair 18.

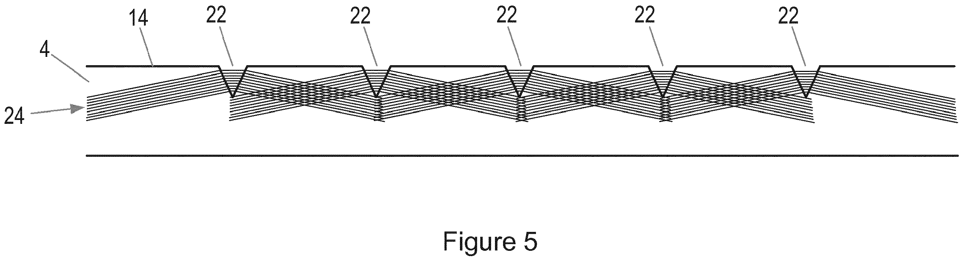

Fig. 5 shows, schematically, a sectional view of the optical waveguide 4 (i.e. through its optical axis), the cutting face 14 having the structure as shown in Fig. 4 above. In this embodiment, the structure of the cutting face 14 comprises the plurality of indentations 22 formed linearly. However, in other embodiments, the indentations 22 may be arranged in some other manner, and the cutting face 14 may be structured in some other way, as is discussed below.

Exemplary paths of light beams 24 propagating through the optical waveguide 4 are shown in Fig. 5. It should be noted that, in the example shown in Fig. 5, the optical waveguide is a silica optical fibre, and only light propagating at the maximum angle supported by such a waveguide (i.e. having a numerical aperture (NA) of 0.22) is shown. Other light may propagate through the optical waveguide 4 at angles smaller than the maximum angle. Typically, when a light beam encounters a sidewall of the optical waveguide 4, it is reflected through the occurrence of total internal reflection, as discussed above. However, when a light beam 24 encounters an indentation 22 formed in the cutting face 14, it is caused to couple out through the wall of the indentation into the surrounding medium.

By forming the indentations 22 as shown in Figs. 4 and 5, each indentation acts as a prism, and serves to refract light incident on its wall. The indentations 22 formed in the waveguide 4 are structured such that the minimum deflection angle (i.e. the angle through which light is refracted by a surface of an indentation) corresponds to the total internal reflection angle of the optical waveguide 4. In other words, each indentation 22 is structured such that, when light propagating through the optical waveguide 4 encounters a sidewall of the indentation, it is refracted to such an extent that, in the surrounding medium, the light travels in direction substantially parallel to the optical axis of the optical waveguide. Accordingly, when the light encounters an opposing sidewall of the indentation 22, it is refracted by a similar angle, causing the light to couple from the surrounding medium back into the optical waveguide 4. Moreover, any light incident on the wall of an indentation 22 at angles higher than the maximum supported angle at which the light 24 in Fig. 5 is shown to be propagating (i.e. light entering the optical waveguide 4 more parallel to the optical axis of the optical waveguide 4) will be refracted to a greater extent, such that, after the each pass through the walls of an indentation 22, the angle of incidence of the light becomes progressively smaller until, eventually, all of the light has an angle of incidence equal to the maximum supported angle (i.e. as shown in Fig. 5).

In one example, the optical waveguide 4 comprises an optical fibre formed from quartz or fused silica, and has a numerical aperture (NA) of 0.22. In such a waveguide, to achieve the effect described above (i.e. the light is deflected towards the maximum support angle of incidence), each indentation 22 is formed as a V-shaped indentation, forming a prism having an apex (i.e. the vertex at the bottom of the indentation, opposite to the opening of the indentation), with an apex angle selected based on the material from which the optical waveguide 4 is formed and the medium surrounding the optical waveguide). A suitable apex angle can be calculated for a given optical waveguide 4 in a given surrounding medium from the following expression:

$$\varphi = \frac{360}{\pi} \frac{NA}{\left(n_1 - n_0\right)},$$

where $\varphi$ is the apex angle in degrees, NA is the numerical aperture of light entering the optical waveguide, $n_1$ is the refractive index of the optical waveguide, and $n_0$ is the refractive index of the surrounding medium.

The table below shows examples of apex angles of indentations for optical waveguides of different materials in different surrounding media (air and water), and with different apertures, calculated using the above expression, for light having a wavelength of 450 nm.

| NA | $n_1$ | $n_0$ | $\varphi$ |
|------|------------------|-----------|------|
| 0.22 | 1.51 (BK7) | 1 (air) | 49° |
| 0.22 | 1.78 (sapphire) | 1 (air) | 32° |
| 0.22 | 1,47 (quartz) | 1 (air) | 54° |
| 0.11 | 1.47 (quartz) | 1 (air) | 27° |
| 0.22 | 1.78 (sapphire) | 1.3 (water) | 52° |
| 0.11 | 1.47 (quartz) | 1.3 (water) | 74° |

In general, therefore, the apex angle may be between approximately 20 degrees and approximately 80 degrees. For a dry shaving scenario (e.g. when the surrounding medium is air) the apex angle may be between approximately 20 degrees and approximately 60 degrees and, and for a wet shaving scenario (e.g. when the surrounding medium is water), the apex angle may be between approximately 45 degrees and approximately 80 degrees. It will be appreciated that, while the above values have been given for surrounding media including air and water, other surrounding media may be used, such as gels and/or foam. In such cases, the apex angle of each indentation may be chosen using the above expression.

In other examples, the indentations may be cone-shaped indentations, or axicons, or may be wedge-shaped indentations. In some embodiments, the indentations may comprise channels formed in the cutting face 14 of the optical waveguide 4. The optical waveguide may, of course, be formed from any other suitable material and, for other materials, the apex angle of the indentations 22 may be different. In general, however, regardless of the material from which the optical waveguide 4 is formed, the indentations have apex angles of between around 20 degrees and around 80 degrees.

In some embodiments, the indentations may be micro-indentations (i.e. indentations having a width in the order of $10^{-6}$ metres) and, in other embodiments, the indentations may be nano-indentations (i.e. indentations having a width in the order of $10^{-9}$ metres). In some examples, the indentations have a width of greater than approximately 25 micrometres ($\mu$m). An indentation having a width of at least 25 $\mu$m is large enough to receive a strand of hair.

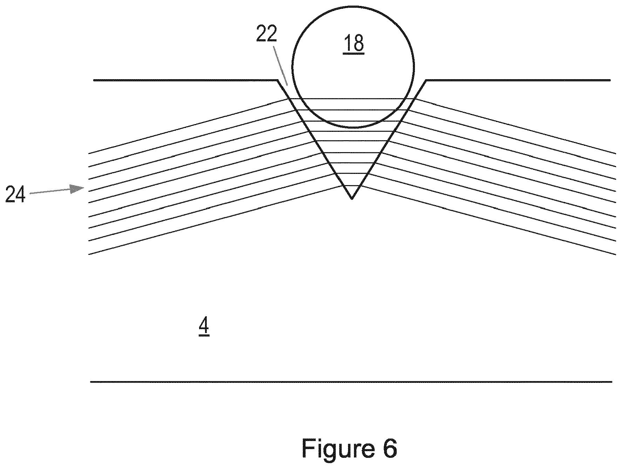

An enlarged view of one of the indentations 22 is shown in Fig. 6, along with a hair 18 to be cut by the cutting device. As discussed above, if an object, such as the strand of hair 18, has a refractive index higher than the optical waveguide 4 and is put into contact with the optical waveguide 4, then frustrated total internal reflection occurs, and light propagating through the optical waveguide can couple into the hair. The intensity of the light that has coupled out of the optical waveguide 4 and which is travelling through the surrounding medium in the indentation 22 is sufficient to initiate melting or breaking of the hair 18. Thus, when the light it brought into contact with the hair 18 (for example, as the shaving device is moved over the skin towards the hairs), before the hair is able to contact the sidewall, the hair begins to deform and melt. Melting of the hair causes an increase in the area of contact between the hair and the cutting face 14 (which includes the walls of the indentation 22) and, consequently, the amount of light able to couple into the hair 18 from the optical waveguide 4 through frustrated total internal reflection increases, resulting in more effective cutting of the hair.

While Figs. 4 and 5 show only five indentations 22 spaced apart along the cutting face 14 of the optical waveguide 4, it will be appreciated that any number of indentations may be included. In some examples, the structure of the cutting face 14 of the optical waveguide 4 may include tens, hundreds or even thousands of indentations arranges in a spaced apart manner, or adjacent to one another. As such, the cutting face 14 of the optical waveguide may have a serrated structure.

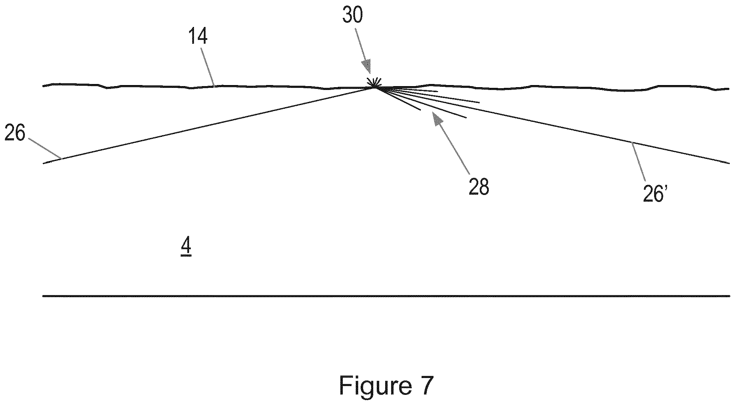

In an alternative embodiment, the structure of the cutting face 14 may comprise a rough or uneven surface instead of a plurality of regular indentations. Such an uneven surface may be formed by applying a controlled roughness to the cutting face 14 of the optical waveguide 4, for example in the form of a Fresnel lens. An uneven surface on the cutting face 14 of the waveguide 4 causes a small amount of the light incident on an inner surface of the cutting face to couple out through the cutting face into the surrounding medium (for example air, or hair if close enough to the cutting face). Fig. 7 is a sectional view of an example optical waveguide 4 (i.e. looking down through the waveguide) having a cutting face 14 which has a structure formed as a rough or uneven surface. In some embodiments, the rough or uneven cutting face may comprise a plurality of small indentations and protrusions. It should be noted that the structure shown in Fig. 7 is greatly exaggerated for clarity. The indentations and protrusions that form the uneven surface of Fig. 7, whether formed as a regular pattern or randomly, may have a width and/or depth or around $10^{-9}$ m (i.e. nanometres) or $10^{-6}$ m (i.e. micrometres). Each indentation and protrusion may have a respective depth and height less than the wavelength of the light intended to propagate through the optical waveguide.

An example light beam 26 is shown propagating through the optical waveguide 4 (from left to right in Fig. 7) at an angle within the range of supported angles (i.e. within the numerical aperture of the optical waveguide). With a rough or uneven cutting face as described above, the majority of the light 26 propagating through the optical waveguide 4 reflects from the internal surface of the cutting face 14, and continues to propagate in the reflected direction (i.e. as light beam 26'), while a small proportion of the light (denoted by label 28) is reflected and scattered in a cone around the point of reflection back into the optical waveguide 4. However, some of the light (denoted by label 30) couples out through the surface of the optical waveguide 4 and is scattered. If a hair is close to the cutting surface 14 of the optical waveguide 4, then the out-coupled light 30 may couple into the hair and initiate cutting or melting of the hair. As with the embodiment discussed above with reference to Figs. 4, 5 and 6, once the hair has begun to melt or deform, the surface area able to contact the cutting surface increases, thereby increasing the effectiveness of the frustrated total internal reflection effect and, consequently, increasing the effectiveness of the hair cutting ability of the shaving device.

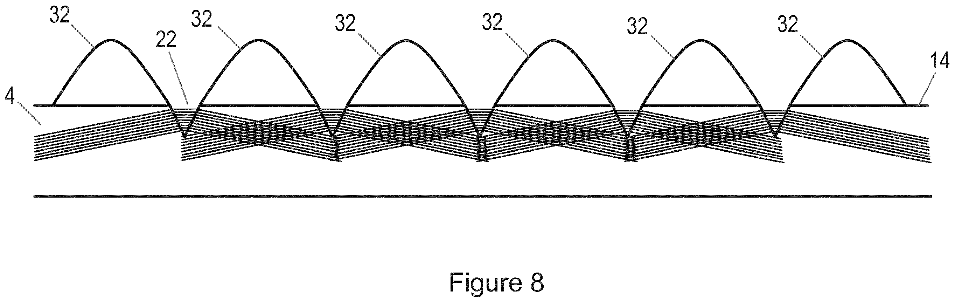

The cutting element may, in some embodiments further comprise means for manipulating or guiding hair before the hair is affected by light coupling out from the optical waveguide 4. Fig. 8 shows one example of such guidance means. The optical waveguide 4 shown in Fig. 8 includes a plurality of protrusions 32 which, in this embodiment, have rounded tops and which serve to guide hairs towards and into the indentations 22 formed in the cutting face 14. In some embodiments, a protrusion 32 may be formed between each adjacent pair of indentations while, in other embodiments, the cutting element may be provided with more or fewer protrusions. Furthermore, the shape of the protrusions 32 shown in Fig. 8 is exemplary, and the protrusions may, in other examples, have any other shape suitable for directing or guiding hair towards the indentations 22 formed in the cutting face 14.

Protrusions 32 may also be formed on the cutting face 14 of the optical waveguide 4 shown in Fig. 7. Again, the protrusions may be shaped and positioned in order to direct hairs towards particular areas of the cutting face 14 so as to increase the effectiveness of the cutting of hairs by the cutting element 4.

The protrusions 32 may, in some embodiments, be formed as an integral part of the optical waveguide 4. In other embodiments, the protrusions may form part of a supporting structure for supporting the optical waveguide 4 on the cutting device 2. In some embodiments, the protrusions 32 may further manipulate the skin from which the hairs to be cut are growing, so as the push hairs out the skin to cause an improved closeness in the shaving or hair cutting activity.

In some examples, at least some of the protrusions 32 may have a width and/or a depth in the order of a few tens of microns ($10^{-6}$ metres).

Therefore, the optical waveguide 4 described above improves the cutting efficiency by initiating the cutting of hairs before frustrated total internal reflection causes total cutting of the hairs. As a result, the cutting of hair by the optical waveguide is more effective, and can reduce the need to move the cutting device repeatedly over the same portion of hair.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cutting element for use in a hair cutting device, the cutting element comprising:
   an optical waveguide having a sidewall which is substantially parallel to the longitudinal axis of the optical waveguide, wherein a portion of the sidewall forms a cutting face for contacting hair, wherein the cutting face has a structure comprising a plurality of indentations configured to allow light to couple out through the sidewall to increase the amount of light able to couple to hair.

2. A cutting element according to claim 1, wherein the structure of the cutting face is such that at least some of the light allowed to couple out through the sidewall is directed back into the optical waveguide.

3. A cutting element according to claim 1 or claim 2, wherein the structure of the cutting face is configured to increase the numerical aperture of light propagating through the optical waveguide.

4. A cutting element according to any of the preceding claims, wherein the structure of the cutting face comprises a serrated structure.

5. A cutting element according to any of the preceding claims, wherein each of the plurality of indentations is configured such that light coupling out through a first wall of the indentation is refracted towards a second wall of the indentation.

6. A cutting element according to any of the preceding claims, wherein each of the indentations comprises one of a wedge-shaped indentation, a V-shaped indentation a cone-shaped indentation, or a channel.

7. A cutting element according to any of the preceding claims, wherein each indentation is configured to receive at least a portion of a strand of hair within the indentation.

**8.** A cutting element according to any of the preceding claims, wherein each indentation comprises an apex having an apex angle of between approximately 20 and 80 degrees.

**9.** A cutting element according to any of the preceding claims, wherein a refraction angle of light out-coupling from the indentation is approximately equal to a total internal reflection angle of the optical waveguide.

**10.** A cutting element according to any of claims 1 to 5, wherein the indentations are micro-indentations or nano-indentations.

**11.** A cutting element according to any of claims 1 to 3, wherein the structure of the cutting face comprises a rough surface, such that a portion of the light propagating through the optical waveguide and incident on the cutting face is caused to couple out through the cutting face.

**12.** A cutting element according to claim 11, wherein the rough surface further comprises a plurality of protrusions, each indentation and protrusion having a respective depth and height less than the wavelength of the light intended to propagate through the optical waveguide.

**13.** A cutting element according to any of the preceding claims, wherein the optical waveguide is an optical fibre.

**14.** A cutting element according to any of the preceding claims, further comprising a plurality of protrusions formed on the cutting face of the optical waveguide, the protrusions configured to guide hair towards the cutting face.

**15.** A hair cutting device for cutting hair on a body of a subject, the hair cutting device comprising:

a light source for generating laser light at one or more specific wavelengths corresponding to wavelengths absorbed by one or more chromophores in hair; and
a cutting element coupled to the light source to receive laser light, the cutting element comprising a cutting element according to any of the preceding claims.

**Patentansprüche**

**1.** Schneidelement zur Verwendung in einer Haarschneidevorrichtung, wobei das Schneidelement umfasst:
einen Lichtwellenleiter mit einer Seitenwand, die im Wesentlichen parallel zur Längsachse des Lichtwellenleiters ist, wobei ein Abschnitt der Seitenwand eine Schneidfläche zum Kontaktieren von Haar bildet, wobei die Schneidfläche eine Struktur aufweist, die eine Vielzahl von Vertiefungen umfasst, die konfiguriert sind, um Licht durch die Seitenwand auskoppeln zu lassen, um die Lichtmenge zu erhöhen, die sich an Haar koppeln kann.

**2.** Schneidelement nach Anspruch 1, wobei die Struktur der Schneidfläche derart ist, dass mindestens ein Teil des Lichts, das durch die Seitenwand auskoppeln kann, wieder zurück in den Lichtwellenleiter geleitet wird.

**3.** Schneidelement nach Anspruch 1 oder Anspruch 2, wobei die Struktur der Schneidfläche konfiguriert ist, um die numerische Apertur des Lichts, das sich durch den Lichtwellenleiter ausbreitet, zu vergrößern.

**4.** Schneidelement nach einem der vorstehenden Ansprüche, wobei die Struktur der Schneidfläche eine gezahnte Struktur umfasst.

**5.** Schneidelement nach einem der vorstehenden Ansprüche, wobei jede der Vielzahl von Vertiefungen so konfiguriert ist, dass das durch eine erste Wand der Vertiefung auskoppelnde Licht zu einer zweiten Wand der Vertiefung hin gebrochen wird.

**6.** Schneidelement nach einem der vorstehenden Ansprüche, wobei jede der Vertiefungen eine der folgenden umfasst: eine keilförmige Vertiefung, eine V-förmige Vertiefung, eine kegelförmige Vertiefung oder einen Kanal.

**7.** Schneidelement nach einem der vorstehenden Ansprüche, wobei jede Vertiefung konfiguriert ist, um mindestens einen Teil einer Haarsträhne innerhalb der Vertiefung aufzunehmen.

**8.** Schneidelement nach einem der vorstehenden Ansprüche, worin jede Vertiefung einen Scheitelpunkt mit einem Scheitelwinkel zwischen etwa 20 und 80 Grad umfasst.

**9.** Schneidelement nach einem der vorstehenden Ansprüche, wobei ein Brechungswinkel der Lichtauskopplung aus der Vertiefung ungefähr gleich einem gesamten inneren Reflexionswinkel des Lichtwellenleiters ist.

**10.** Schneidelement nach einem der Ansprüche 1 bis 5, wobei die Vertiefungen Mikrovertiefungen oder Nanovertiefungen sind.

**11.** Schneidelement nach einem der Ansprüche 1 bis 3, wobei die Struktur der Schneidfläche eine raue Oberfläche umfasst, so dass ein Teil des Lichts, das sich durch den Lichtwellenleiter ausbreitet und auf die Schneidfläche auftrifft, durch die Schneidfläche ausgekoppelt wird.

12. Schneidelement nach Anspruch 11, wobei die raue Oberfläche weiter eine Vielzahl von Vorsprüngen umfasst, wobei jede Vertiefung und jeder Vorsprung eine entsprechende Tiefe und Höhe aufweist, die kleiner als die Wellenlänge des Lichts ist, das sich durch den Lichtwellenleiter ausbreiten soll.

13. Schneidelement nach einem der vorstehenden Ansprüche, wobei der Lichtwellenleiter eine Lichtleitfaser ist.

14. Schneidelement nach einem der vorstehenden Ansprüche, weiter umfassend eine Vielzahl von Vorsprüngen, die auf der Schneidfläche des Lichtwellenleiters ausgebildet sind, wobei die Vorsprünge konfiguriert sind, um Haar zur Schneidfläche hin zu führen.

15. Haarschneidevorrichtung zum Schneiden von Haaren auf einem Körper eines Probanden, wobei die Haarschneidevorrichtung umfasst:

eine Lichtquelle zum Erzeugen von Laserlicht bei einer oder mehreren spezifischen Wellenlängen, die den Wellenlängen entsprechen, die von einem oder mehreren Chromophoren im Haar absorbiert werden; und
ein Schneidelement, das mit der Lichtquelle gekoppelt ist, um Laserlicht zu empfangen, wobei das Schneidelement ein Schneidelement gemäß einem der vorstehenden Ansprüche umfasst.

**Revendications**

1. Elément de coupe destiné à être utilisé dans un dispositif de coupe de cheveux, l'élément de coupe comprenant :
un guide d'onde optique ayant une paroi latérale qui est sensiblement parallèle à l'axe longitudinal du guide d'onde optique, dans lequel une partie de la paroi latérale forme une face de coupe destinée à entrer en contact avec les cheveux, dans lequel la face de coupe a une structure comprenant une pluralité d'indentations configurées pour permettre à la lumière d'être couplée à travers la paroi latérale pour augmenter la quantité de lumière pouvant être couplée aux cheveux.

2. Elément de coupe selon la revendication 1, dans lequel la structure de la face de coupe est telle qu'au moins une partie de la lumière pouvant être couplée à travers la paroi latérale est redirigée dans le guide d'onde optique.

3. Elément de coupe selon la revendication 1 ou la revendication 2, dans lequel la structure de la face de coupe est configurée pour augmenter l'ouverture numérique de la lumière se propageant à travers le guide d'onde optique.

4. Elément de coupe selon l'une quelconque des revendications précédentes, dans lequel la structure de la face de coupe comprend une structure dentelée.

5. Elément de coupe selon l'une quelconque des revendications précédentes, dans lequel chacune de la pluralité des indentations est configurée de telle sorte que la lumière couplée à travers une première paroi de l'indentation est réfractée vers une seconde paroi de l'indentation.

6. Elément de coupe selon l'une quelconque des revendications précédentes, dans lequel chacune des indentations comprend l'une parmi une indentation cunéiforme, une indentation en forme de V, une indentation conique, ou un canal.

7. Elément de coupe selon l'une quelconque des revendications précédentes, dans lequel chaque indentation est configurée pour recevoir au moins une partie d'une mèche de cheveux dans l'indentation.

8. Elément de coupe selon l'une quelconque des revendications précédentes, dans lequel chaque indentation comprend un apex ayant un angle d'apex entre environ 20 et 80 degrés.

9. Elément de coupe selon l'une quelconque des revendications précédentes, dans lequel un angle de réfraction de la lumière se découplant de l'indentation est à peu près égal à un angle de réflexion interne total du guide d'onde optique.

10. Elément de coupe selon l'une quelconque des revendications 1 à 5, dans lequel les indentations sont des micro-indentations ou des nano-indentations.

11. Elément de coupe selon l'une quelconque des revendications 1 à 3, dans lequel la structure de la face de coupe comprend une surface rugueuse, de sorte qu'une partie de la lumière se propageant à travers le guide d'onde optique et incidente à la surface de coupe est découplée à travers la face de coupe.

12. Elément de coupe selon la revendication 11, dans lequel la surface rugueuse comprend en outre une pluralité de proéminences, chaque indentation et proéminence ayant une profondeur et une hauteur respective inférieure à la longueur d'onde de la lumière destinée à se propager à travers le guide d'onde optique.

13. Elément de coupe selon l'une quelconque des revendications précédentes, dans lequel le guide d'onde optique est une fibre optique.

14. Elément de coupe selon l'une quelconque des revendications précédentes, comprenant en outre une pluralité de proéminences formées sur la face de coupe du guide d'onde optique, les proéminences étant configurées pour guider les cheveux vers la face de coupe.

15. Dispositif de coupe de cheveux pour couper des cheveux sur un corps d'un sujet, le dispositif de coupe de cheveux comprenant :

une source de lumière pour générer une lumière laser à une ou plusieurs longueurs d'ondes spécifiques correspondant à des longueurs d'onde absorbées par un ou plusieurs chromophores dans les cheveux ; et
un élément de coupe couplé à la source de lumière pour recevoir une lumière laser, l'élément de coupe comprenant un élément de coupe selon l'une quelconque des revendications précédentes.

Figure 1

Figure 2

Figure 3

Figure 4

Figure 5

EP 3 500 201 B1

Figure 6

Figure 7

Figure 8

**EP 3 500 201 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014143670 A

### Non-patent literature cited in the description

- **M. D. GREENWELL ; A. WILLNER ; PAUL L. KIRK.** Human Hair Studies: III. Refractive Index of Crown Hair. *31 Am. Inst. Crim. L. & Criminology,* 1940, vol. 746